# EUROPEAN PATENT APPLICATION

(11) **EP 2 272 554 A1**
(43) Date of publication of application: **12.01.2011**
(21) Application number: 09164045.8
(22) Date of filing: 29.06.2009
(51) Int. Cl.: A61M 5/158

(54) **Inserter Device**

(71) Applicant: Unomedical A/S, 3460 Birkerød (DK)
(72) Inventor: Gyrn, Steffen, 4100, Ringsted (DK); Thorup, Sören, 2700, Brønshøj (DK); Vikkelsö, Thomas Heinstrup, 2500, Valby (DK)
(74) Representative: Nilausen, Kim

(57) **Abstract**

The application concerns an insertion device for inserting a medical device or a part of medical device into the subcutaneous or intramuscular area of a patient.
An inserter device according to the application comprises
- a housing (1);
- a cover (2) which is slidably connected to the housing (1) and has at least one retracted and one forward position relative to the housing (1);
- a hub (5) for an insertion needle (4) which can be moved linearly relative to the housing (1) and relative to the cover (2);
- a primary spring (6) which moves the hub (5) for the insertion needle (4) relative to the cover (2).
The primary spring (6) is configured to pull the hub (5) for the insertion needle (4) either to a forward or to a retracted position.

## Description

### Technical field of the invention

The invention concerns an insertion device for inserting a medical device or a part of medical device into the subcutaneous or intramuscular area of a patient.

### Background of the invention

An inserter device also called inserter or injector is commonly used in the medical field for inserting medical devices, such as infusion sets, sensors or the like, through the skin of a patient in a more or less automated fashion.

Commonly, when using an inserter, the user, i.e. the patient or the treatment provider e.g. nurse, doctor, relative, or the like has to apply a force towards the surface of the skin of the patient in order to provide injection of the medical device or a part of the medical device having the form of a needle, a cannula, a sensor, or the like. This can cause physiological or psychological distress and/or discomfort, and may lead to inappropriate application of the medical device. Many people are afraid of sharp objects, such as injection needles and other penetrating devices, commonly used for medical treatment and therapy. This fear is often irrational, and it may hamper an appropriate medical treatment. For example in the case of self-medication, a lack of administration of an appropriate dose of a required medical composition can lead to complications, which may even be life-threatening. When treating diabetes, e.g. in juveniles, there is a risk that the required insulin-dose may not be self-administered due to irrational fear of the insertion needle, combined with a general lack of knowledge and awareness concerning the consequences of omitting the correct application of the device and dosage.

A further known issue with insertion of medical devices is the risk of contamination of the penetrating member before or during application. This can easily lead to the introduction of an infection to a patient, e.g. through a contaminated insertion needle. The longer such a needle is exposed, the higher the risk of accidental contamination, e.g. by touching the needle with a finger, bringing the needle in contact with an unclean surface, or by airborne contamination, aerosol contamination and the like. Depending on the nature of the contamination (e.g. comprising virus, bacteria, fungus, yeast and/or prion) combined with the general health status of the patient, the resulting infection can rapidly turn into a life threatening situation.

Finally, it is well known that contact with an infected, used needle especially in hospital environments can be life-threatening, and the risk of accidental exposure to contaminated material in the form of a used insertion needle must be minimized.

Thus, there is an obvious need in the art for a robust, reliable, accurate, safe, hygienic, and user friendly insertion device, which addresses the issues discussed above.

### Summary of the invention

The object of the invention is to provide an inserter device comprising
- a housing (1);
- a cover (2) which is slidably connected to the housing (1) and has at least one retracted and one forward position relative to the housing (1);
- a hub (5) for an insertion needle (4) which can be moved linearly relative to the housing (1) and relative to the cover (2);
- a primary spring (6) which moves the hub (5) for the insertion needle (4) relative to the cover (2);
   wherein the primary spring (6) is configured to pull the hub (5) for the insertion needle (4) either to a forward or to a retracted position.

A primary spring which pulls the needle hub is loaded by being stretched and released by being retracted i.e. reducing the distance between the "two ends" of the spring, where the two "ends" might just represent two different positions of the spring. It is advantageous to use pulling springs as these springs are less expensive and very robust.

According to one embodiment the inserter device comprises a secondary spring (7). The secondary spring can either have the purpose of retracting the needle hub i.e. bring the needle hub from a forward to a retracted position in an inserter having automatic insertion and retraction, or to produce a resistance for an inserter only having automatic retraction, in this case the secondary spring is used to dimension the pressure used to insert the insertion needle subcutaneously during manual insertion, or to restore the inserter device to a certain length. The second spring can e.g. be a spiral spring made of plastic or metal.

According to one embodiment the primary spring (6) is releasably attached to the upper end of the cover (2) at a first end and at the second end it is indirectly connected to the housing (1) in such a way that the second end of the primary spring (6) can be displaced by rotating the housing (1) relative to the cover (2).

According to one embodiment the primary spring (6) comprises at least one elastically mounted arm (19) which arm (19) at the upper end is provided with an outward hook (20) securing the arm (19) to the cover (2) by catching around and upper edge of the cover (2). When the hook is pushed inwards i.e. towards the central part of the inserter, the arm is released from the upper edge. The primary spring (6) can comprise two elastically mounted arms (19) which arms (19) at the upper end each are provided with an outward hook (20).

According to one embodiment an inclined surface (11) of the housing through contact with a surface of the primary spring (6) or of a part (9) in a fixed connection with the primary spring (6) forces the second end of the primary spring (6) away from upper end of the cover (2).

According to one embodiment the primary spring (6) is releasably attached to the upper end of the cover (2) at a first end and at the second end it is indirectly connected to the housing (1) in such a way that the second end of the primary spring (6) can be displaced by pressing the housing (1) and the cover (2) towards each other along the longitudinal axis of the two parts. The primary spring (6) can at the upper end be provided with an outward hook (20) securing the upper end of the primary (6) to the cover (2) by catching around and upper edge of the cover (2). When the hook is pushed inwards i.e. towards the central part of the inserter, the arm is released from the upper edge.

According to one embodiment a central part of the housing through contact with a surface of the second end of the primary spring (6) or of a part (9) in a fixed connection with the second end of the primary spring (6) forces the second end of the primary spring (6) away from upper end of the cover (2).

According to one embodiment the housing (1) comprises at least one protruding part (30) which upon contact with the upper end of the primary spring (6) connects unreleasably to the upper end of the primary spring (6).

According to one embodiment a second spring (7) is positioned between the second end of the primary spring (6) and the lower end of the cover (2).

According to one embodiment the primary spring (6) is constituted by an elastic ring-shaped band which at one end is attached to the upper end of the cover (2) and at the second end is attached to the needle hub (5).

According to one embodiment a part of the housing (1) is in contact with the needle hub (5) and this part can move relative to the cover in a direction along the longitudinal axis of the cover (2) thereby moving the needle hub (5) relative to the cover (2) and pushing the needle hub (5) towards the skin of the patient. The part of the housing (1) in contact with the needle hub (5) can be constituted by two flexible arms (40) provided with hooks which hooks are in contact with an upper surface of the needle hub (5).

According to one embodiment the medical device is divided into a subcutaneously placed part and a site where the site comprises means for releasably attaching and connecting a connector part and means for attaching the site to the surface of the patients skin. The connector part might either be a part through which fluid is delivered to an infusion device or it might be a signalling part which can receive and send signals to/from a sensor

According to one embodiment the housing (1) and the lid (12) are each constituted of a hard material and form an outer shell completely surrounding the inner parts and providing sterile conditions before separation of the two parts, where the housing (1) and the lid (12) each are provided with at least one inclined contact surface (11, 13) which contact surfaces slide along each other when the housing (1) is rotated relative to the lid (12) causing the housing (1) and the lid (12) to be separated from each other in a direction parallel to the longitudinal axis of the inserter device. The housing (1) can comprise an internal inclined surface (10) which slide against a part of or a part connected to the primary spring (6) during rotation of the housing (1) relative to the lid (12) which forces the primary spring (6) into a loaded state.

### Brief description of the drawings

A detailed description of embodiments of the current invention will be made with reference to the accompanying figures, wherein like numerals designate corresponding parts in different figures.
Fig. 1 shows an exploded view of a first embodiment of an inserter device according to the invention, this inserter has automatic insertion and automatic retraction of the insertion needle.
Figs. 2A-B show the first embodiment of the inserter device respectively in a ship state and in a loaded state.
Fig. 3A-C shows the first embodiment of the inserter device respectively in a state where the insertion needle is fully inserted and in a state where the insertion needle has been retracted to a fully retracted position, and details of the release of the needle hub from the trigger plate.
Fig. 4 shows an exploded view of a second embodiment of an inserter device according to the invention, this inserter has manual insertion and automatic retraction of the insertion needle.
Figs. 5A-B show the second embodiment of the inserter device in two states; first in a state before insertion and second in a state where the insertion needle is fully inserted.
Fig. 6 shows details of the second embodiment of the inserter device.
Fig. 7 shows the second embodiment of the inserter device in a state where the insertion needle is fully retracted and the cover is distanced as much as possible from the housing.
Fig. 8A-D show a third embodiment of an inserter device according to the invention. The third embodiment is shown in four states: A: ship state; B: just before loading; C: insertion needle fully inserted; D: insertion needle fully retracted.
Fig. 9 show the position of the rubber band used with the third embodiment of the inserter device.
Fig. 10 shows the first embodiment of the inserter device in a ship state.
Fig. 11 shows a lid and a corresponding cover according to the first embodiment of the inserter device.
Fig. 12 shows an enlargement of a needle hub used with the first and the second embodiment.
Fig. 13A and B shows an embodiment of the housing and fig. 13C shows an embodiment of the cover.

### Detailed description of the invention

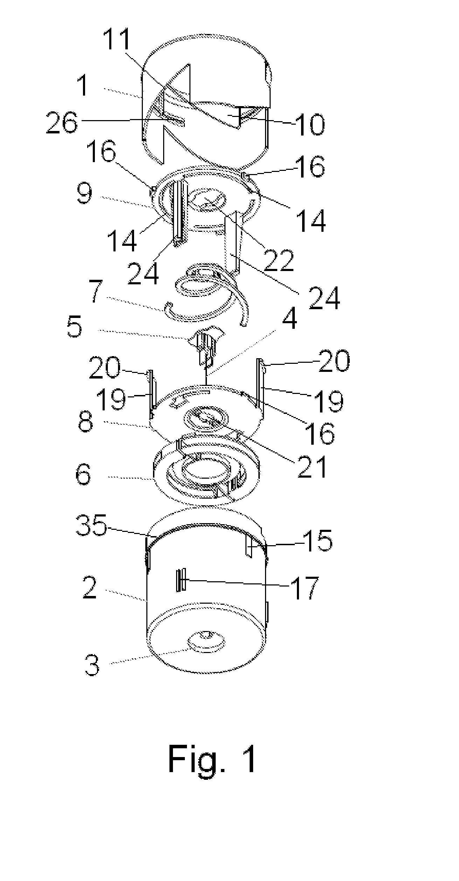

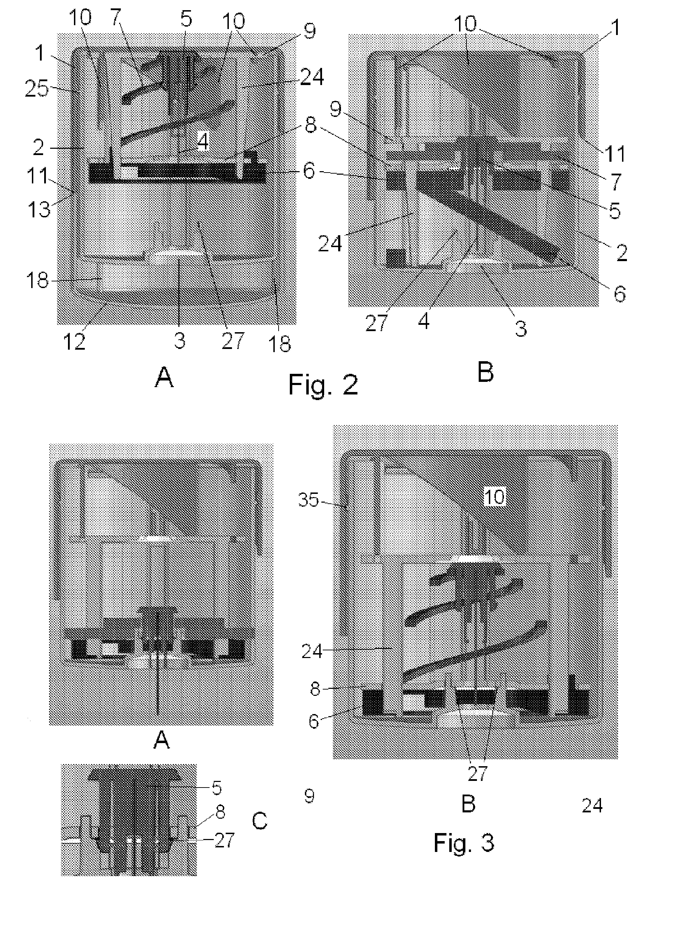

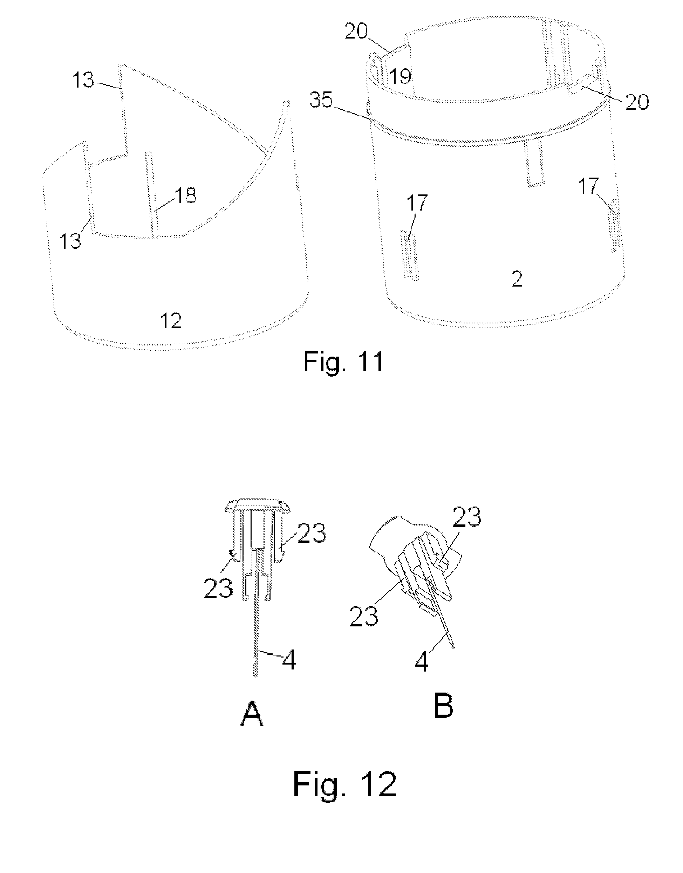

Fig. 1 shows a first embodiment of an inserter device. The embodiment has automatic insertion and automatic retraction of the insertion needle and comprises a housing 1, a cover 2, an insertion needle 4 attached to a needle hub 5, a primary spring 6 used for subcutaneous insertion of the insertion needle 4, a secondary spring 7 used for retraction of the insertion needle 4, a trigger plate 8 and a spring loader 9 i.e. this embodiment comprises 7 separate pieces, 8 including a lid 12 (see fig. 2A and 11)" and all of the pieces are normally made of moulded plastic except e.g. the insertion needle 4 which might be made of metal.

The housing 1 is provided with one or more internal wings 10 having a rounded profile and an inclined surface facing the direction of rotation of the housing 1. That the profile is rounded means that the length of the internal wing 10 follows the direction of rotation when the housing 1 is rotated around the longitudinal central axis of the housing 1. The first embodiment is provided with three of these internal wings 10 which wings 10 are attached to or moulded as part of the internal top surface of the housing 1.

Also the outer wall of the housing 1 is provided with inclined contact surfaces 11 facing the direction of rotation of the housing at the time where the housing 1 is released from the lid 12. The lid 12 provides a complete cover of the internal parts of the inserter device during shipment and thereby makes it possible to keep the internal parts sterile before use. According to the first embodiment three inclined contact surfaces 11 form part of the outer wall of the housing 1 defining three contact surfaces. The lid 12 is also provided with inclined contact surfaces 13 (see fig. 2A and 11) facing the direction of rotation of the lid 12 at the time where the housing 1 is s released from the lid 12.

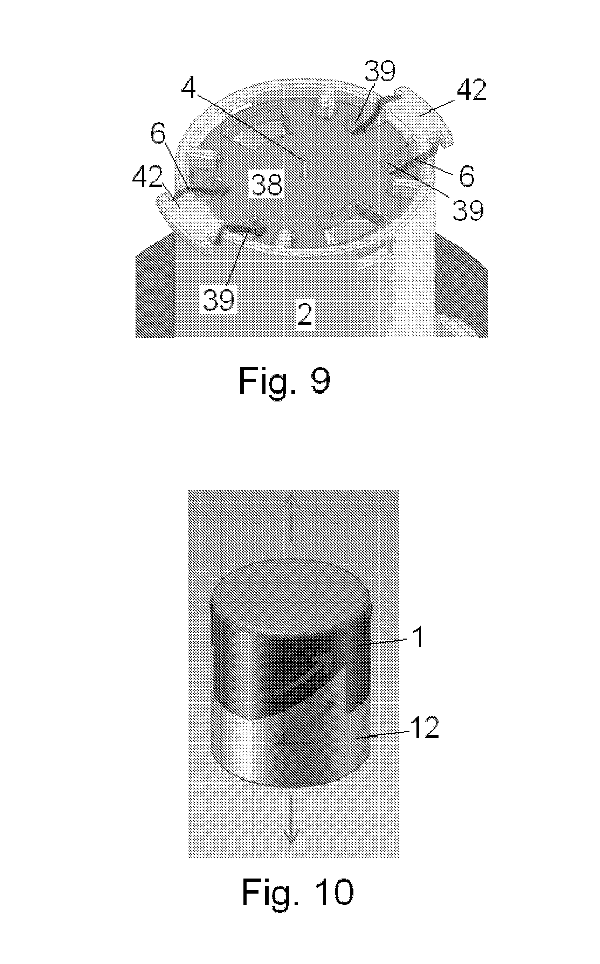

The inserter device is loaded as the lid 12 is removed by rotating the lid 12 relative to the housing 1. The inclined contact surfaces 11 and 13 respectively of the housing 1 and of the lid 12, slide against each other as the lid 12 is rotated/removed whereby the lid 12 and the housing 1 are forced in opposite directions along the longitudinal axis of the inserter device (illustrated in fig. 10).

The cover 2 is locked longitudinally to the housing 1 by longitudinal locking means. According to the first embodiment of the inserter device the longitudinal locking means comprise a protruding edge 35 provided on the top half of the cover 2 extending perpendicularly to the longitudinal axis of the inserter device on the outer surface of the cover 2, and a corresponding groove 26 extending on the inner surface of the housing 1.

The cover 2 is also locked rotationally relative to the lid 12 and therefore the cover 2 will rotate together with the lid 12. According to the present embodiment the cover 2 is rotationally locked to the lid 12 by rotational locking means comprising three longitudinal grooves 17 ("longitudinal" means that the grooves 17 are parallel to the longitudinal central axis of the inserter device) provided on the outer surface of the cover 2 which grooves 17 each correspond to a longitudinal protruding part 18 (see fig. 2A and 11) on the lid 12. The rotation of the lid 12 causes the inclined surface of each internal wing 10 of the housing 1 to be pushed against a surface of the spring loader 9, according to the first embodiment the spring loader 9 is provided with a number of wing openings 14 corresponding to the number of internal wings 10 of the housing 1 and each internal wing 10 of the housing pushes against an inner surface of such an opening 14. As the contact surface of each internal wing 10 is inclined and as the spring loader 9 is kept in position by the elastic pressure of the secondary spring 7, the pressure against the inner surface of the opening 14 forces the spring loader 9 away from the top of the housing 1.

Both the spring loader 9 and the trigger plate 8 are locked rotationally relative to the cover 2 by rotational locking means, i.e. both parts rotate together with the cover 2. According to the first embodiment of the inserter device the locking means comprises three peripheral protruding parts 15 of respectively the spring loader 9 and the trigger plate 8 fitting into three corresponding longitudinal tracks 16 on the inner surface of the cover 2.

The trigger plate 8 is attached to the housing 1 close to the top of the housing 1 by to flexible arms 19 provided with hooks 20. The two oppositely positioned flexible arms 19 keep the trigger plate 8 in a position close to the housing top as the hooks 20 catch around an upper surface of the housing 1. According to the first embodiment of the inserter device this upper surface is provided by two recesses in the upper edge of the housing where the positions of the recesses correspond to the position of the hooks 20. When the two oppositely positioned flexible arms 19 are pushed out of the recess the trigger plate is forced forward by the primary spring 6 if the inserter device is in a loaded state.

The trigger plate 8 also has a central opening 21 through which the insertion needle 4 of the needle hub 5 extends. The central opening 21 is though too small for the needle hub 5 to pass through so the needle hub 5 is retained by the trigger plate 8.

The primary spring 6 is placed between the trigger plate 8 and the bottom of the cover 2 where the bottom is considered to be the end of the cover to which the insertion needle 4 points and where the infusion site is positioned 3 if the infusion site is separated from the subcutaneously inserted part. The primary spring 6 is at one end unreleasably attached to the trigger plate 8 and at another end the primary spring 6 is attached to protruding legs 24 of the spring loader 9. During the rotation of the housing 1 relative to the lid 12, the inserter device is loaded by extending the primary spring 6 by forcing the spring loader 9 forward. One end of the primary spring 6 is attached to the protruding legs 24 of the spring loader 9 while the second end of the primary spring 6 is attached to the stationary trigger plate 8. The trigger plate 8 is stationary relative to the housing 1 during loading of the springs. Actually, two ends of the primary spring 6 is fastened to the protruding legs 24 of the spring loader 9 according to the first embodiment as this embodiment of the primary spring 6 comprises two half circles of moulded plastic where one end of each half circle has to be displaced in order to load the primary spring 6. As the primary spring 6 is forced into a loaded state by being extended, it will attempt to return to the unloaded state by contracting.

The secondary spring 7 is forced into a loaded state when it is compressed and it will attempt to return to an unloaded state by extending. The secondary spring 7 is placed between the spring loader 9 and the trigger plate 8, and the secondary spring 7 is loaded when the lid 12 is rotated relative to the housing 1 and then removed whereby the spring loader 9 is pressed against the trigger plate 8. Finally the lid 12 is removed from the housing 1 by this rotating action as the lid 12 and the housing 1 are pushed in opposite directions along the longitudinal axis of the inserter device during the rotation. According to the first embodiment the secondary spring 7 is a spiral spring made of plastic.

The needle hub 5 is before loading and until the springs 6 and 7 reach the fully loaded state attached to the spring loader 9. The spring loader 9 has a central opening 22 into which opening 22 the top of the needle hub 5 fits from the lower side i.e. the side which is facing the infusion site. The opening 22 is too small for the needle hub 5 to pass through, and as the secondary spring 7 pushes upward on the needle hub 5 i.e. toward the spring loader 9, the secondary spring 7 keeps the needle hub 5 in the correct position relative to the spring loader 9.

The needle hub 5 is provided with locking means 23 locking the needle hub 5 to the trigger plate 8 in the longitudinal direction when the needle hub 5 is pushed sufficiently through the central opening 21. According to the first embodiment of the inserter device the locking means 23 have the form of two oppositely positioned flexible arms each provided with an outward hook at the end, the outer surface of each hook having an inclined or rounded surface (see fig. 12).

The cover 2 comprises release means 27 for releasing the needle hub 5 from the trigger plate 8 after full insertion of the insertion needle 4. The release means 27 comprises two upright parts which extend from the lowest surface of the cover 2, the two upright parts correspond to the locking means 23 of the needle hub 5 and when the locking means 23 of the needle hub 5 are pressed against the release means 27 the needle hub 5 is released from the trigger plate 8, and the needle hub 5 can be pushed to a retracted position by the secondary spring 7.

The first embodiment of the inserter device according to the invention functions as illustrated in the figures 2 and 3.

Fig. 2A shows the inserter device in the ship state i.e. the state which the device is in during storage i.e. before use. The primary spring 6 is in a completely unloaded state and the secondary spring 7 is in an almost unloaded state. Normally, it is advantageous that the springs are in an unloaded or almost unloaded state during storage as this will keep the springs from deteriorating functionally during storage. The spring loader 9 rests against the top wall of the housing 1 and is prevented from changing position by the secondary spring 7. The lid 12 is mounted onto the cover 2 resting against the inclined contact surfaces 11 of the housing 1. The hooks 20 of the flexible arms 19 of the trigger plate 8 rest in the recesses along the upper edge of the housing and keep the trigger plate in the correct pre-trigger position. An infusion site is positioned at the position 3 for the infusion site and a subcutaneous part is loaded onto the insertion needle 4 of the needle hub 5. All parts inside the closed room formed by the housing 1 and the lid 12 have been sterilised after manufacture of the inserter device.

When a user wants to use the inserter device, the user will first remove the lid 12 by rotating the lid 12 relative to the housing 1. This action not only removes the lid 12 from the device but also loads the inserter device simultaneously. Normally the two parts, housing 1 and lid 12, will be manufactured in bright and easily distinguishable colours which will make it easy for the user to identify the direction of rotation for each part due to the inclined corresponding surfaces (see fig. 10).

Fig. 2B shows the inserter device in a loaded state. Both the primary spring 6 and the secondary spring 7 are in a fully loaded state. The spring loader 9 is now positioned at the outermost end of the internal wings 10. The lid 12 has been removed. The hooks 20 of the flexible arms 19 of the trigger plate 8 still rest in the recesses along the upper edge of the housing and keep the trigger plate in the correct pre-actuated position. The locking means 23 of the needle hub have been locked to the trigger plate 8 i.e. the trigger plate 8 and the needle hub 5 will now move together. The infusion site is still positioned at the position 3 for the infusion site and a subcutaneous part is still loaded onto the insertion needle 4 of the needle hub 5. Non-sterilised air has to some degree access to the inner room formed by the cover 2 and the housing 1 and the user will therefore have to use the inserter device within a short time period.

When the user has prepared the inserter device for insertion by removing the lid 12, the user places the device against the skin of the patient whether this might be the user himself or a second person. According to the shown embodiment the infusion site is separated from the subcutaneously positioned part and placed in the position 3. Normally an adhesive surface of the infusion site is exposed which adhesive surface is used to attach the infusion site releasably to the patients skin. The adhesive surface could be exposed automatically upon removal of the lid 12 or it could be exposed manually e.g. by removing a release paper from the adhesive surface before use. When the adhesive surface is exposed the end of the inserter device comprising the infusion site is pushed against the skin of the patient, and then the trigger is activated. The trigger according to the shown embodiment is activated by pressing two opposite flexible points at the top end of the housing 1. This pressure releases the two flexible arms 19 of the trigger plate 8 and the trigger plate 8 is then no longer held in the stationary position at the top of the housing 1 as the primary spring 6 is attached unreleasably to the trigger plate 8 and will force the trigger plate 8 to a new stationary position at the bottom of the cover 2.

Fig. 3A shows the inserter device in an unstable state where the insertion needle 4 is inserted subcutaneously. The primary spring 6 is unloaded and the secondary spring 7 is in a fully loaded state. The spring loader 9 is still positioned at the outermost end of the internal wings 10. The hooks 20 of the flexible arms 19 of the trigger plate 8 are released from the upper edge of the housing and the trigger plate 8 is moved to the end position at the bottom of the cover 2. The subcutaneously part has been inserted subcutaneously and is now unreleasably attached to the infusion site; the infusion site is still positioned at the position 3. The locking means 23 of the needle hub 5 have just gotten into contact with the release means 27 of the housing 1, and the needle hub 5 is no longer attached to the trigger plate 8.

Fig. 3B shows the inserter device in the final state where the needle hub 5 has been retracted from the infusion site. Both the primary and the secondary springs 6 and 7 are unloaded. The secondary spring 7 has pushed the needle hub 5 to the retracted position as soon as the needle hub 5 has been released from the trigger plate 8. The secondary spring 7 is placed between the needle hub 5 and the trigger plate 8 thereby providing a force pushing these two parts away form each other.

Fig. 3C shows in detail how the release of the needle hub 5 from the trigger plate is performed. An edge of the stationary release means 27 forces the arms provided with outward hooks of the needle hub 5 inwards, and this action causes the needle hub 5 to be released from the trigger plate 8.

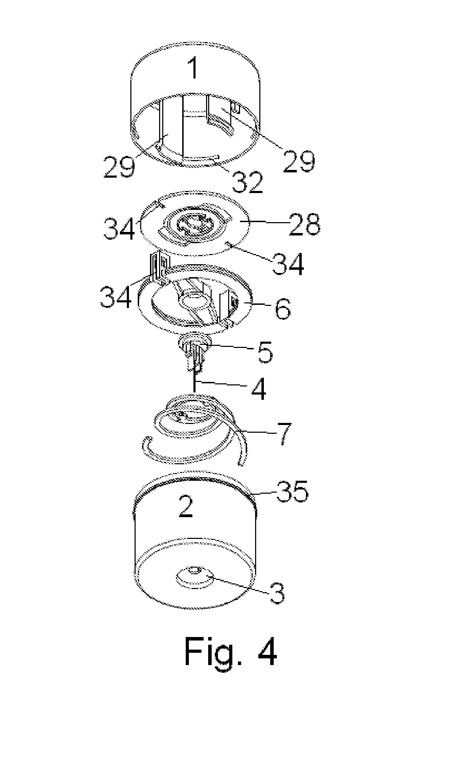

Fig. 4 shows a second embodiment of an inserter device. Parts similar to parts of the first embodiment are illustrated with the same reference numbers as used when describing the first embodiment e.g. the needle hub 5, the primary spring 6, the secondary spring 7 and the release means 27 for the needle hub 5 are identical to the corresponding parts of the first embodiment. The second embodiment has manual insertion and automatic retraction of the insertion needle and comprises a housing 1, a cover 2, an insertion needle 4 attached to a needle hub 5, a primary spring 6 used for retraction of the insertion needle 4, a secondary spring 7 used to provide a comfortable resistance when manually inserting the insertion needle, a needle base plate 28 and a not shown lid i.e. this embodiment comprises 7 separate pieces and all of the pieces are normally made of moulded plastic except e.g. the insertion needle 4 which might be made of metal.

As the first embodiment of the inserter device, the internal parts of the device are held in a sterile environment as long as the not shown lid is attached to the housing 1 and encloses the cover 2.

The housing 1 comprises two extending parts 29 extending from the closed top surface of the housing 1. The two extending parts 29 are slightly flexible i.e. they can pivot relative to the position where they are fixed, this flexibility is only used during manufacturing of the device. The housing 1 also comprises two pairs of catchers 30 which catchers 30 are formed as oppositely facing hooks and each pair of catchers 30 can catch and hold on to hole 31 at one end of the primary spring 6. Also the housing 1 comprises a series of elevations 32 close to the lower edge, the elevations 32 might be elongated and in a direction perpendicular to the longitudinal axis of the device or they might be formed as points. The elevations 32 prevent the cover 2 from sliding out of the housing 1 i.e. the cover can move longitudinally relative to the housing 1 but only to a certain degree as the elevations 32 meet restriction e.g. in form of protrusions 35 on the outer surface of the cover 2.

The cover 2 comprises a position 3 for an infusion site at the bottom of the cover 2 and release means 27 positioned on top of the position for the infusion site of the same form as the release means 27 of the first embodiment. Further the cover 2 comprises two upright walls 33 protruding opposite each other from the side surface of the cover 2. The upright walls 33 extend from the top of the cover 2 to the bottom and correspond to peripheral openings 34 in the needle base plate 28 allowing the needle base plate 28 to slide in the longitudinal direction of the cover 2 along the upright walls 33. The cover 2 also comprises the circular protrusion 35 extending from the outer surface of the cover 2 near the top end.

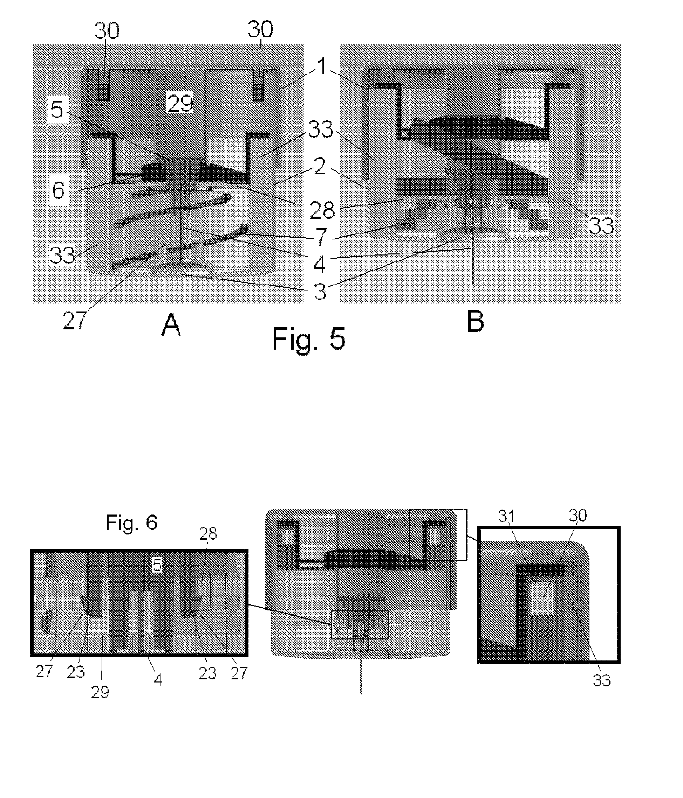

Fig. 5A shows the inserter device in the ship state i.e. an unloaded state which the device is in during storage i.e. before use. The primary spring 6 is unloaded and the secondary spring 7 is almost unloaded. That the secondary spring 7 is almost unloaded means that it exercises a slight pressure against the surfaces between which it is placed. The needle hub 5 extends through central openings in both the primary spring 6 and the needle base plate 28. The needle hub 5 is inserted through the central openings during manufacturing of the device and as the top of the needle hub 5 is provided with an increased or increasing diameter, the needle hub 5 cannot pass through the central opening of the primary spring 6. Also the needle hub 5 comprises locking means 23 of the same form as illustrated for the first embodiment and these locking means 23 catch around the lower surface of the needle base plate 28 and lock the primary spring 6 and the needle base plate 28 together. The primary spring 6 is at this state attached to the top of the cover 2 as two protruding parts at each their end of a flexible half circle spring are hooked around the top of an upright wall 33 of the cover 2. The needle base plate 28 is locked to the end of the extending parts 29 of the housing 1. The secondary spring 7 pushes the bottom of the cover 2 away from the needle base plate 28 and thereby upholds the length of the device.

When a user wants to start using the inserter device, the not shown cylindrical lid is first removed. Any release liner covering the adhesive distal surface of the infusion site is released. Then the user places the device with the bottom of the cover 2 against the patients skin and pushes the housing toward the skin of the patient.

Fig. 5B shows the inserter device in a loaded state - just before release of the needle hub 5 - where user has pushed the housing 1 toward the patients skin, and the insertion needle 4 has been fully inserted. The primary spring 6 has been extended and thereby loaded. As the central part of the primary spring 6 is locked between the needle hub 5 and the needle base plate 28, the primary spring 6 is forced into the extended state as the housing 1 and thereby the extending parts 29 are pushed down. The secondary spring 7 is loaded and the user has to overcome the resistance provided by the secondary spring 7 during loading. When the housing 1 is in the position of this state the catchers 30 extending from the top surface of the housing 1 will have pivoted away from each other i.e. outwards as the protruding parts of the primary spring 6 comprising the holes 31 are squeezed in between the two hooks of the catchers 30. The action of the catchers 30 returning to the inward position causes a click-sound and indicates to the user that the insertion needle 4 has been fully inserted, and as the click sounds one end of the primary spring 6 is locked to the top surface of the housing 1 in stead of being hooked around the top of the upright walls 33 of the cover 2.

As the needle hub 5 is pressed against the release means 27, the needle hub 5 is released from the needle base plate 28 as the arms of the locking means 23 are pushed inward i.e. toward each other, and the hooks of the locking means 23 release the grip of the needle base plate 28.

When one end of the primary spring 6 is attached to the catchers 30 of the top surface of the housing 1 and the other end is no longer locked to the needle base plate 28, the primary spring 6 can return to the unloaded state by contracting, and this contraction causes the withdrawal of the needle hub 5 to a retracted position near the top of the housing 1.

Fig. 6 shows in detail in the left view how the needle hub 5 is released from the needle base plate 28 when the locking means 23 in the form of two oppositely flexible arms with outward hooks are pressed together as a result of the interaction with the release means 27 which are in the form of two upright protrusions of a hard material and the inclined outer surfaces of the two hooks.
The release of the needle hub 5 takes place when the insertion needle 4 has been inserted to such a depth i.e. the housing 1 has been pushed forward to such a degree that the outward hooks of the flexible arms touches the release means 27. Also another detail is shown in the right view, this view shows how the upper end of the primary spring 6 which is provided with a hole 31 hooks around the upright wall 33 of the cover 2. At the position shown in fig. 6 the housing 1 and the cover 2 are pressed together to the outmost degree, i.e. the combination of the housing 1 and cover 2 is as short as possible.

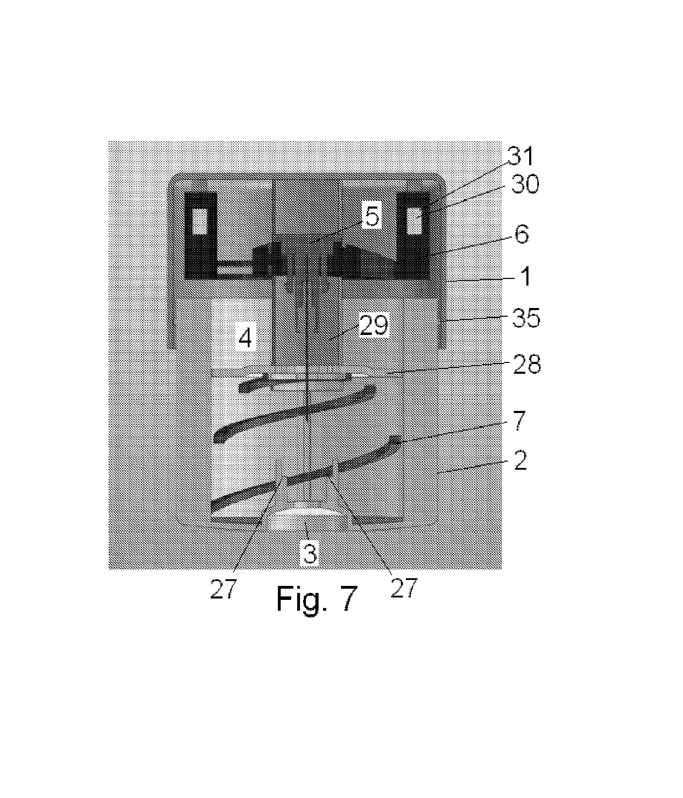

Fig. 7 shows the second embodiment of the inserter in an unloaded state obtained after insertion of the insertion needle. The primary spring 6 is in a completely unloaded state and the secondary spring 7 is in an almost unloaded state, the secondary spring 7 is providing a force big enough to keep the cover 2 and the housing 1 away from each other in such a degree that the assembled two parts i.e. housing 1 and cover 2, will be as long as possible, and the length is determined by the elevations 32 of the housing 1 getting in contact with the circular protrusion 35 of the cover 2.

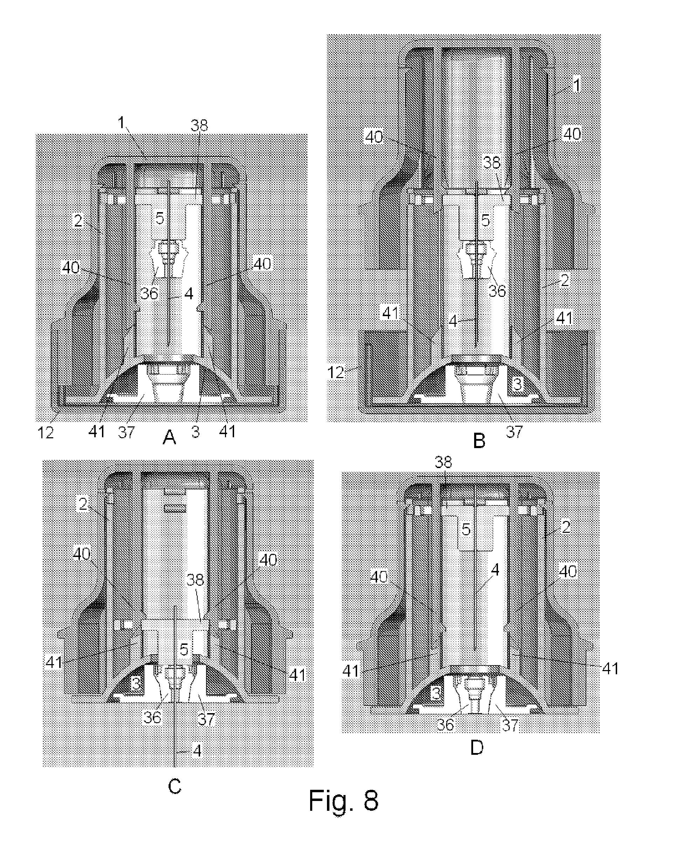

Fig. 8 and 9 illustrate a third embodiment of an inserter according to the present invention. Parts similar to parts of the first and second embodiments are illustrated with the same reference numbers as used when describing the two former embodiments e.g. the needle hub 5, the primary spring 6, and the release means 27 for the needle hub 5 are similar to the corresponding parts of the first embodiment. The third embodiment has manual insertion and automatic retraction of the insertion needle. This embodiment comprises 5 separate pieces and all of the pieces are normally made of moulded plastic except the primary spring 6 which is e.g. made of soft flexible rubber band and e.g. the insertion needle 4 which might be made of metal.

The figs. 8A-8D also shows a two part infusion device comprising a subcutaneous part 36 and an infusion site 37. The subcutaneous part 36 comprises a part which is to be inserted subcutaneously in the patient and is preloaded onto the insertion needle 4 and the infusion site 37 is attached to the cover 2 inside the position 3 for infusion site. The inserter device might also be used for insertion of other medical devices comprising a subcutaneous part whether such a device would be in one or more pieces, and then the position 3 for the infusion site would have to be adapted to the medical device in question.

Fig. 8A shows the third embodiment of the inserter device in a ship state which is an unloaded state. As illustrated in fig. 9, the primary spring 6 which according to this embodiment can be constituted by a single circular rubber band is attached to the top of the cover 2 and supports under the needle hub 5 i.e. the primary spring 6 pulls the needle hub 5 and the top of cover 2 together. The needle hub 5 of this embodiment is constructed with a circular top plate 38 having a diameter small enough to allow the needle hub 5 to slide up and down inside the cylindrical cover 2. The circular top plate 38 has openings 39 allowing for the rubber band constituting the primary spring 6 to pass from the lower side of the needle hub 5 to the top of the cover 2 without by its presence disturbing the sliding movement of the needle hub 5 relative to the cover 2. According to the shown embodiment the rubber band 6 is attached to the top of the cover 2 by winding it around a protruding part of the edge 42, this is easily done as the rubber band is very flexible. The circular top plate 38 is also provided with two openings and a flexible loading arm 40 provided with an inward hook extend through each opening. The inserter device is in fig. 8A provided with a lid 12 and the lid 12 assures sterile conditions for the internal parts of the inserter device before opening.

Fig. 8B shows the third embodiment of the inserter device in a pre-loaded state, the primary spring 6 (not shown) is unloaded in this state. The lid 12 has been released from the housing 1 and the housing 1 has been pulled back relative to the cover 2. This action causes the inward hooks of the two flexible loading arms 40 to move to the upper side of the circular top plate 38 of the needle hub 5, and due to the flexibility of the loading arms 40 the hooks are caught on top of the needle hub 5. When the device has been put into this state the device is ready for insertion when the lid 12 has been removed from the device. Normally removal of the lid 12 will lead to that a protective layer such as a release layer is removed from the adhesive side of the medical device placed in the position 3 for the infusion site.

Fig. 8C shows the third embodiment of the inserter device in a fully loaded state where the insertion needle 4 is also fully inserted. The primary spring 6 is fully loaded in this state as the rubber band which is attached to the top of the cover 2 has been stretched to its maximum as the needle hub 5, which the rubber band is placed under, is at its lowest position. In this position the release means 41 get in contact with the flexible loading arms 40 and push the loading arms 40 outward i.e. away from each other. This movement causes the needle hub 5 to be released from the hooks of the loading arms 40 which result in the needle hub 5 being pulled upwards to the top of the cover 2 where the opposite ends of the circular rubber band are fastened.

Fig. 8D shows the third embodiment of the inserter device in the final unloaded state where the needle hub 5 and thereby the insertion needle 4 has been brought into the retracted position. The state is similar to the unloaded state shown in fig. 8A except that the two part infusion device 36, 37 is now assembled and the subcutaneous part 36 is inserted into the infusion site 37.

Fig. 9 show a possible position of a primary spring 6 in the form of an elastic such as a rubber band used with the third embodiment of the inserter device. The elastic band is attached to the top of the cover 2 by wrapping parts of the elastic band around protruding parts 42 extending outward from the upper edge of the cover 2. The central plate being encompassed by the wall of the cover 2 is a circular top plate 38 of the needle hub 5 and the elastic band passes through openings 39 provided in the top of the needle hub 5 which openings are large enough to prevent the elastic band from interfering to interfere with the movement of the needle hub 5 relative to the cover 2.

Fig. 10 shows the first embodiment of the inserter device in a ship state. The housing 1 is dark (green) and the lid 12 is light (grey). The difference in colour provides the user with a clear impression of which direction to twist the two parts relatively. The two central arrows of the figure illustrates the direction of rotation of the housing 1 relative to the lid 12, and the two arrows at each end of the device illustrate the direction of movement of respectively the housing 1 and the lid 12 upon rotation. The inclined surfaces 13 and 11 respectively of the lid 12 and the housing 1 provide a robust guidance for the rotation/separation of the two parts i.e. the user only has to focus on the rotation as the linear separation of the two parts is an unavoidable consequence of the rotation. The rotation also causes the device to be loaded which result in that the user only has to perform one act i.e. rotate the lid 12 relative to the housing 1 and then the device is in a ready-to-use state.

Fig. 11 shows a lid and a corresponding cover according to the first embodiment of the inserter device.

Fig. 12 shows an enlargement of a needle hub used with the first and the second embodiment of the inserter device. The needle hub 5 has two flexible arms comprising locking means 23 in the form of outward hooks. Also, the needle hub 5 comprises a top part which is used to attach the needle hub 5 to e.g. the trigger plate 8 of the first embodiment.

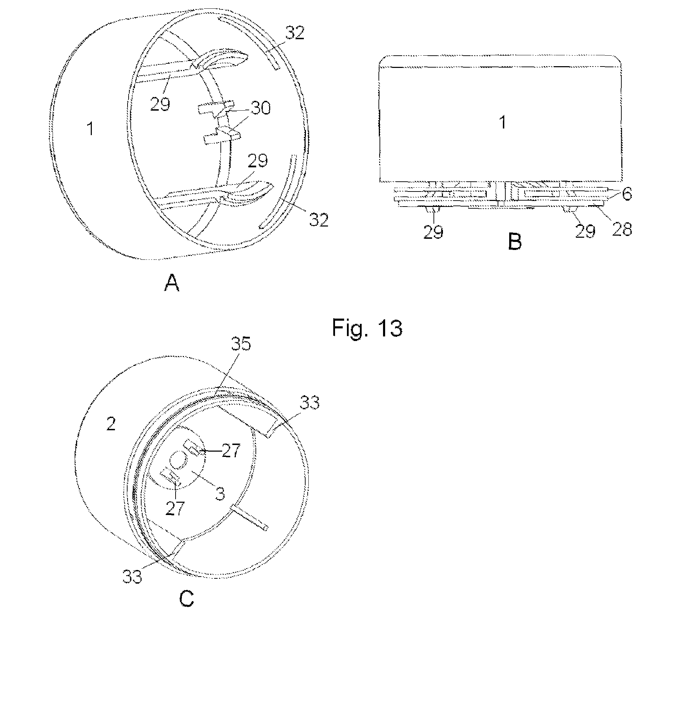

Fig. 13A and B shows embodiments of a housing to be used with the first embodiment and fig. 13C shows an embodiment of a cover to be used with the first embodiment.

## Claims

1. An inserter device for inserting a medical device subcutaneously which inserter device comprises
- a housing (1);
- a cover (2) which is slidably connected to the housing (1) and has at least one retracted and one forward position relative to the housing (1);
- a hub (5) for an insertion needle (4) which can be moved linearly relative to the housing (1) and relative to the cover (2);
- a primary spring (6) which moves the hub (5) for the insertion needle (4) relative to the cover (2);
**characterized in that** the primary spring (6) is configured to pull the hub (5) for the insertion needle (4) either to a forward or to a retracted position.

2. An inserter device according to claim 1, wherein the inserter device comprises a secondary spring (7).

3. An inserter device according to claim 2, wherein the primary spring (6) is releasably attached to the upper end of the cover (2) at a first end and at the second end it is indirectly connected to the housing (1) in such a way that the second end of the primary spring (6) can be displaced by rotating the housing (1) relative to the cover (2).

4. An inserter device according to claim 3, wherein the primary spring (6) comprises at least one elastically mounted arm (19) which arm (19) at the upper end is provided with an outward hook (20) securing the arm (19) to the cover (2) by catching around and upper edge of the cover (2).

5. An inserter device according to claim 3 or 4, wherein an inclined surface (11) of the housing through contact with a surface of the primary spring (6) or of a part (9) in a fixed connection with the primary spring (6) forces the second end of the primary spring (6) away from upper end of the cover (2).

6. An inserter device according to claim 1 or 2, wherein the primary spring (6) is releasably attached to the upper end of the cover (2) at a first end and at the second end it is indirectly connected to the housing (1) in such a way that the second end of the primary spring (6) can be displaced by pressing the housing (1) and the cover (2) towards each other along the longitudinal axis of the two parts.

7. An inserter device according to claim 6, wherein the primary spring (6) at the upper end is provided with an outward hook (20) securing the upper end of the primary (6) to the cover (2) by catching around and upper edge of the cover (2).

8. An inserter device according to claim 6 or 7, wherein a central part of the housing through contact with a surface of the second end of the primary spring (6) or of a part (9) in a fixed connection with the second end of the primary spring (6) forces the second end of the primary spring (6) away from upper end of the cover (2).

9. An inserter device according to claim 6, 7 or 8, wherein the housing (1) comprises at least one protruding part (30) which upon contact with the upper end of the primary spring (6) connects unreleasably to the upper end of the primary spring (6).

10. An inserter device according to claim 6, 7, 8 or 9 , wherein a second spring (7) is positioned between the second end of the primary spring (6) and the lower end of the cover (2).

11. An inserter device according to claim 1 or 2, wherein the primary spring (6) is constituted by an elastic ring-shaped band which band at one end is attached to the upper end of the cover (2) and at the second end is attached to the needle hub (5).

12. An inserter device according to claim 11, wherein a part of the housing (1) is in contact with the needle hub (5) and this part can move relative to the cover in a direction along the longitudinal axis of the cover (2) thereby moving the needle hub (5) relative to the cover (2) and pushing the needle hub (5) towards the skin of the patient.

13. An inserter device according to claim 12, wherein the part of the housing (1) in contact with the needle hub (5) is constituted by two flexible arms (40) provided with hooks which hooks are in contact with an upper surface of the needle hub (5).

14. An inserter device according to any of the claims 1-7, wherein the housing (1) and the lid (12) each are constituted of a hard material and form an outer shell completely surrounding the inner parts and providing sterile conditions before separation of the two parts, where the housing (1) and the lid (12) each are provided with at least one inclined contact surface (11, 13) which contact surfaces slide along each other when the housing (1) is rotated relative to the lid (12) causing the housing (1) and the lid (12) to be separated from each other in a direction parallel to the longitudinal axis of the inserter device.

15. An inserter device according to claim 14, wherein the housing (1) comprises an internal inclined surface (10) which slide against a part of or a part connected to the primary spring (6) during rotation of the housing (1) relative to the lid (12) which forces the primary spring (6) into a loaded state.
| **Reference no.** | **Part** |
|---|---|
| 1 | Housing |
| 2 | Cover |
| 3 | Position for infusion site |
| 4 | Insertion needle |
| 5 | Needle hub |
| 6 | Primary spring |
| 7 | Secondary spring |
| 8 | Trigger plate |
| 9 | Spring loader |
| 10 | Internal wing of housing |
| 11 | Contact surface of housing |
| 12 | Lid |
| 13 | Contact surface of lid |
| 14 | Wing opening in spring loader |
| 15 | Peripheral protruding parts |
| 16 | Openings in cover |
| 17 | Longitudinal grooves |
| 18 | Longitudinal protruding parts |
| 19 | Flexible arm of trigger plate |
| 20 | Hooks of arms 19 |
| 21 | Central opening in trigger plate 8 |
| 22 | Central opening in spring loader 9 |
| 23 | Locking means of needle hub 5 |
| 24 | Protruding legs of spring loader 9 |
| 25 | Protruding edge on the outer surface of the cover |
| 26 | Corresponding groove on inner surface of the housing |
| 27 | Release means of cover |
| 28 | Needle base plate |
| 29 | Extending parts of housing |
| 30 | Catchers of housing |
| 31 | Hole in primary spring |
| 32 | Elevations of housing |
| 33 | Upright walls of the cover |
| 34 | Peripheral openings |
| 35 | Circular protrusion |
| 36 | Subcutaneous part of infusion device |
| 37 | Infusion site of infusion device |
| 38 | Circular top plate |
| 39 | Openings in circular top plate |
| 40 | flexible loading arm |
| 41 | Release means of third embodiment |
| 42 | Protruding part of the edge |
